# EUROPEAN PATENT APPLICATION

(11) **EP 3 153 576 A1**
(43) Date of publication of application: **12.04.2017**
(21) Application number: 16192714.0
(22) Date of filing: 07.10.2016
(51) Int. Cl.: C12N 5/078, C12N 5/077, C12N 5/074

(54) **IMPROVED EXPANSION OF EUKARYOTIC CELLS**

(30) Priority: 09.10.2015 DE 102015117245
(71) Applicant: RheinCell Therapeutics GmbH, 40225 Düsseldorf (DE)
(72) Inventor: Ströcker, Annika, 40225 Düsseldorf (DE); Heins, Nico, 40764 Langenfeld (DE); Meffert, Andrea, 53225 Bonn (DE); Wernet, Peter, 40627 Düsseldorf (DE)
(74) Representative: Bungartz Christophersen Partnerschaft mbB Patentanwälte

(57) **Abstract**

A process of improving the expansion of eukaryotic cells in culture in an aqueous medium which is contained in a culture vessel comprising a substrate for the eukaryotic cells, the process comprising the steps of:
- providing a lysate obtainable from a thrombocytes containing source,
- incubating the substrate with the lysate for a sufficient time to allow a coating of the substrate with components of the lysate prior to
- contacting the eukaryotic cells with the substrate.

## Description

The present invention pertains to a process of improving the expansion of eukaryotic cells in culture in an aqueous medium which is contained in a culture vessel comprising a substrate for the eukaryotic cells, a process of manufacturing a coated substrate, a coated substrate obtainable by the latter process and the use of the coated substrate in a process of improving the expansion of eukaryotic cells.

### Summary of the invention

The present invention is based on the finding that the presence of substrates which had been in contact with a lysate of thrombocytes is beneficial for the expansion of eukaryotic cells, in particular stem cells. Subject matter of the present invention is a process of improving the expansion of eukaryotic cells in culture in an aqueous medium which is contained in a culture vessel comprising a substrate for the eukaryotic cells, the process comprising the steps of:
- providing a lysate obtainable from a thrombocytes containing source,
- incubating the substrate with the lysate for a sufficient time to allow a coating of the substrate with components of the lysate prior to
- contacting the eukaryotic cells with the substrate.

In an embodiment of the process of the invention the substrate consisting of fibronectin, collagen is provided by an inner surface of the culture vessel, the substrate is a scaffold, a micro-carrier, or a membrane.

In another embodiment of the process of the invention the culture vessel is a flask, bioreactor, tube, scaffold, a micro-carrier, or a membrane.

In yet another embodiment the process of the invention the eukaryotic cells are selected from the group consisting of stem cells, or from stem cells differentiated cells.

In a further embodiment the process of the invention the eukaryotic cells are Induced Pluripotent Stem cells (IPS), human embryonic stem cells, mesenchymal stem cells, human unrestricted fetal stem cells, or any somatic stem cells.

In a still further embodiment the process of the invention the medium is a stem cell or cell culture medium.

In yet another embodiment of the process of the invention the time to allow a coating of the substrate is at least about 10 seconds, in particular about half hour to about two days.

In another embodiment of the process of the invention the components of the lysate comprise substances are selected of the group consisting of fibronectin, fibrin, collagen and laminin. Another subject of the present invention is a process for manufacturing a coated substrate comprising the steps of
- providing the substrate,
- contacting the substrate with a lysate of thrombocytes in a medium sufficient to coat the substrate with components of the lysate of thrombocytes,
- removing the medium.

In one embodiment the process for manufacturing a coated substrate of the invention performs after removing of the medium a finishing step, such as a washing of the resultant product, deep-freezing, freeze-drying or lyophilisation the resultant product.

Still another subject of the present invention is a coated substrate obtainable by the process for manufacturing a coated substrate according to the invention.

In one embodiment the substrate of the invention the substrate is a coated microcarrier, coated scaffold, culture vessel having a coated inner surface, tube, scaffold, a micro-carrier, or a membrane.

In still another embodiment the substrate of the invention the culture vessel having a coated inner surface is selected from the group consisting of a flask, bioreactor, tube, or a membrane.

Subject matter of the invention is also the use of the coated substrate of the invention the process of the invention.

### Detailed description of the invention

### BACKGROUND OF INVENTION

Pluripotent stem cells (hPS) are characterized as cells with high telomerase activity, infinite capacity for self-renewal, and pluripotency (the ability to differentiate into any cell type represented in the three germ layers, ectoderm, endoderm and mesoderm). As a result, a great deal of hope is associated with the potential application of human pluripotent stem cells in the fields of cell therapy and regenerative medicine.

The first successful derivation of human embryonic stem cells (hESC) was reported by Thomson, J. A. et al. (Science (1998). In that study, the cells were isolated from the inner cell mass of blastocysts and plated onto mitotically inactivated murine embryonic fibroblast (MEF) feeder cells. Initial hESC cell derivation and culture has originated from culturing practices developed predominantly for murine ES (mES) cells over the past 25 years. In 2006, Kazutoshi Takahashi and Shinya *Yamanaka* established for the *first time* murine ES-like cell lines (iPSC) from mouse embryonic fibroblasts and skin fibroblasts in the presence of feeder cells. While MEF or human foreskin fibroblast feeders (HFF) have proved to be a robust surface for long term cultures of hPS cells, this methodology has several limitations. First, there are concerns about potential animal pathogens that could be transferred to the human pluripotent stem cells (hES) cells, making them unsuitable for clinical applications. Second, it is inconvenient and tedious to grow and maintain two cells types. Furthermore, it is difficult to transfect and genetically manipulate the compacted hES cell colonies on feeders. For all these reasons, efforts have been initiated to develop feeder-free conditions for culturing hES cells.

Especially the manipulation of specific signaling regulators has facilitated the development of feeder-free conditions required for propagating undifferentiated hES cells. Studies of several groups have clearly delineated that iPSC can be grown in the absence of feeders, as long as they are plated on an appropriate extra-cellular matrix (ECM) and cultured in media either conditioned on feeder cells or supplemented with soluble factors. Even commercialized products are nowadays on the market (Stemcell Tech, etc.).

An ECM is a three-dimensional molecular complex that varies in composition, and may include components such as laminin, fibronectin, collagens and other glycoproteins, hyaluronic acid, proteoglycans, and elastins. Examples of commercially available extracellular matrices include, for example, ECM gel, Vitronectin, Geltrix or Matrigel. Matrigel matrix is a reconstituted basement membrane isolated from mouse Engelbrecht-Holm-Schwarm (EHS) tumor and mainly composed predominantly of laminin, collagen IV, entactin and heparan sulfate proteoglycans. Matrigel matrix also contains a number of growth factors, such as EGF, IGF-1, PDGF, TGF-β, VEGF and bFGF that exhibits substantial *lot-*to*-lot variation.*

For human pluripotent cells (hES), it is known, that they can be propagated in an undifferentiated state on an ECM surface. There are a number of advantages in using an ECM as a feeder-free substrate for culturing hES cells. For instance, DNA, RNA and protein isolation is easier on an ECM surface due to lack of potential contamination from the feeder cells not to mention potential therapeutic applications of the cells. For example, genetic manipulation of hES cells requires efficient transfection capabilities. Transfection efficiencies of hES cells grown on a Matrigel or fibronectin surface are improved compared to those grown on feeders. When cultured without feeder cells, it has been shown that for example hES cells form "monolayer-like" colonies, making individual cells more accessible for cell manipulation techniques.

### OBJECT AND SUMMARY OF THE INVENTION

The present invention provides culturing equipment and methods using such culture vessels (bioreactors) for the stable, consistent propagation of stem cells in a substantially undifferentiated state, without utilizing a feeder layer to support the growth of the cells which is substantially devoid of pathogens. Surprisingly, it has been discovered that, in order to propagate and maintain hES cell and iPS cell cultures in an undifferentiated state, it is not necessary to provide an extracellular matrix coating in an amount sufficient to produce a 3D matrix for the cell. Rather, the extracellular matrix coating only needs to be adsorbed by or bound to the cell vessel surface (tissue culture plastic, microcarrier) in a minimal solution concentration sufficient to provide extracellular matrix proteins at a saturated level in a proper conformation (LIT).

### INVENTION

The invention further provides a cell culture product including a substrate (negatively charged plastic); and a coating thereon deposited from a coating solution. The coating solution includes a mixture of extracellular matrix proteins and an aqueous solvent, wherein the total protein concentration in the coating solution is about 10 μg/ml to about 1 mg/ml or higher.

The cell culture product of the invention can be characterized by the density of extracellular matrix protein(s) adsorbed to the coated surface relative the density of extracellular matrix protein(s) adsorbed to an uncoated, but otherwise identical control surface. In particular, the present invention provides a cell culture product that includes a substrate material defining a surface and a coating deposited on the surface of the substrate, in which the coating includes at least one extracellular matrix protein selected from the following: fibronectin, collagen, laminin, entactin, and heparan sulfate proteoglycans, and the density of the extracellular matrix protein adsorbed to the surface.

Vitrification (or even Lyophilisation): The extracellular matrix coating can be dried, providing a consistent surface containing extracellular matrix components. In one embodiment, the culture vessel is capable of storage at room temperature for an extended period of time. In one embodiment, the culture vessel can be stored at room temperature for at least two weeks.

The present invention also provides a culture vessel for human stem cells including: i) a vessel/microcarrier suitable for culturing cells; ii) a fixed concentration of extracellular matrix components, wherein the extracellular matrix is in a dried state on at least one surface of the container.

Also provided are methods of preparing the cell culture vessels/microcarrier of this invention. In some embodiments, the method includes applying an hPLP matrix coating solution to a surface of a substrate, wherein the total protein concentration in the coating solution is about 10 μg/ml to about 1 mg/ml. The method also includes incubating the coating solution on the substrate surface; removing the incubated coating solution from the substrate; and washing the substrate after the removing step. Finally, the method includes drying the washed substrate.

### Vessel coating (culture flasks etc)

In other embodiments the culturing method includes the steps of a) first applying the hPLP solution to a vessel suitable for cell culturing, wherein the extracellular matrix includes at least laminin, collagen etc; b) incubating the vessel for a period of time sufficient to allow binding of the hPLP matrix with the vessel; c) removing said first solution from the vessel; d) washing the vessel to remove any of the first solution remaining in the vessel; and e) drying the vessel. Further provided are methods of culturing human stem cells. In some embodiments, the method includes providing a cell culture product including a substrate; and a coating thereon deposited from a coating solution. The coating solution includes a mixture of extracellular matrix proteins and an aqueous solvent, the total protein concentration in the coating solution being about 10 μg/ml to about 1 mg/ml. The culturing method also includes adding a suspension of embryonic stem cells and media to the cell culture product; and incubating the embryonic stem cells at 5 % CO₂ in humidified air at 37 °C. to produce undifferentiated colonies for embryonic stem cell expansion.

### Microcarrier (polyurethane etc carrier)

In some other embodiments the method for propagating human stem cells includes the step of a) providing an apparatus for propagating stem cells that includes a microcarrier suitable for culturing cells; and an extracellular matrix in a fixed concentration; wherein the extracellular matrix includes at least laminin, collagen etc; and wherein the extracellular matrix is in a dried state on at least one surface of the microcarrier. This method further includes the steps of b) adding a cell culture medium that has been previously conditioned by an appropriate feeder culture to the microcarrier; and c) adding stem cells to the microcarrier. Moreover, the method includes the step of d) incubating the container including the stem cells under a suitable condition to allow for the propagation of the stem cells in a bioreactor.

### DESCRIPTION OF THE INVENTION

### EXAMPLES

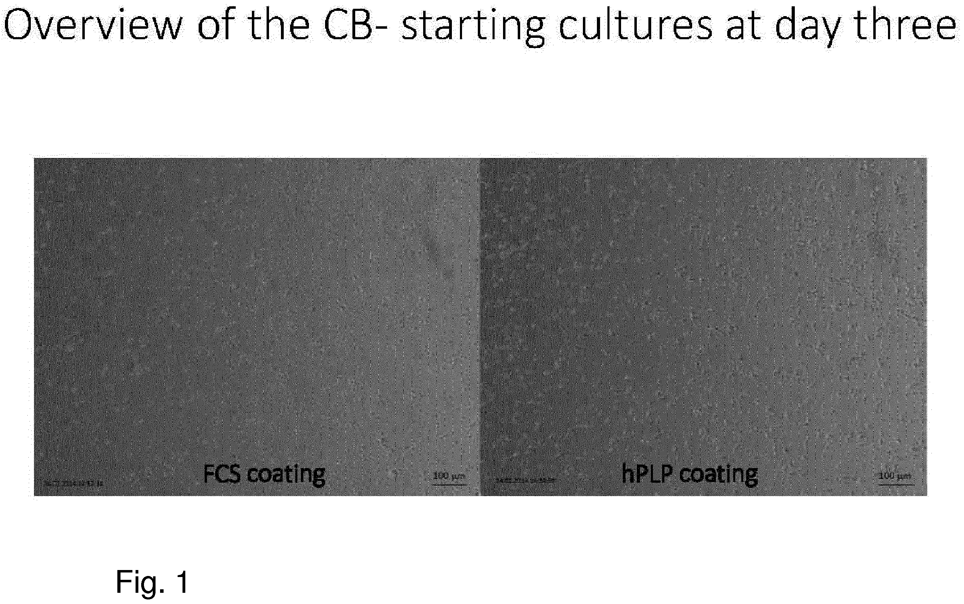

- FIG. 1: shows a phase-contrast microphotograph illustrating the overview of a starting CB-blood culture on a hPLP coating/film compared to FBS coating.

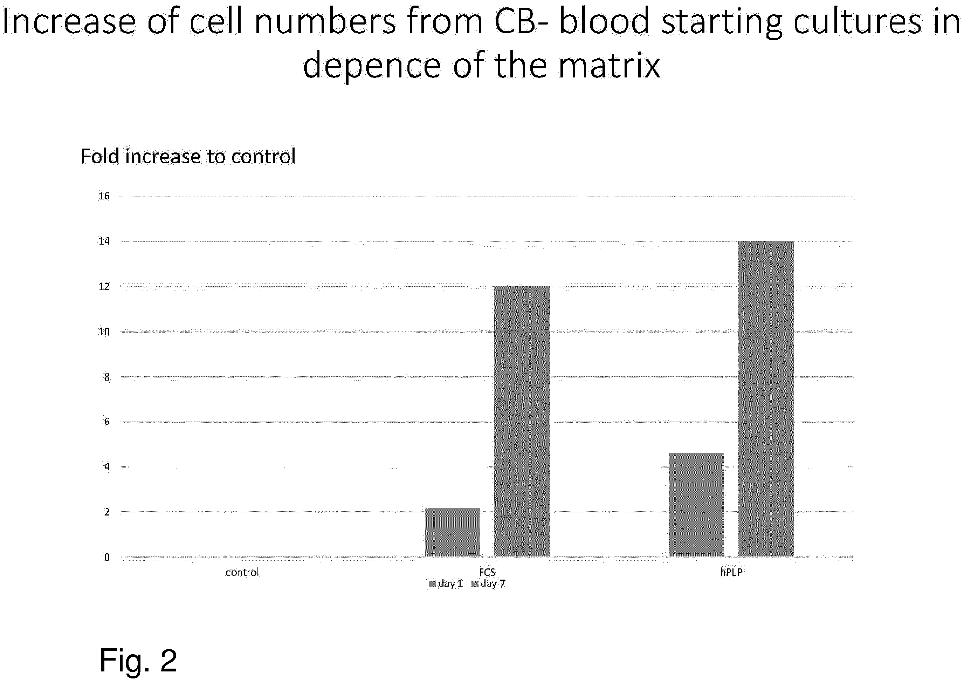

- FIG. 2: shows the cell numbers from CB- blood and fold increase of the cell numbers of MSCs cultured on plastic surface (Ctrl), FBS coating and (FBS) or platelet lysate coating (hPLP) on day 1, day 7 and day 14 after isolation.

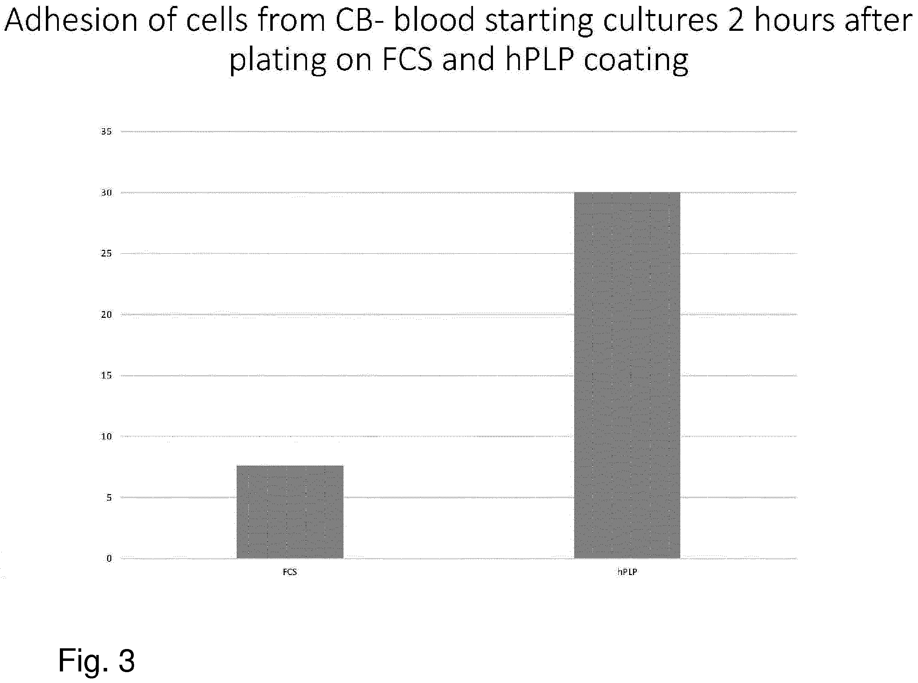

- FIG. 3: shows the dependence of the adhesion of CB-blood cells from the substrate: plastic surface (Ctrl), FBS coating and (FBS) or platelet lysate coating (hPLP). Cell numbers 2h after plating.

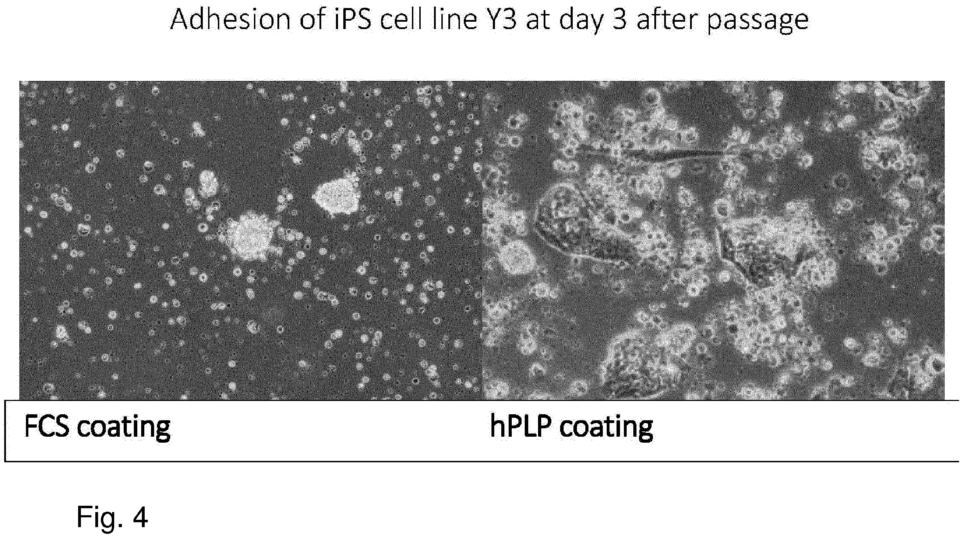

- FIG. 4: shows photographs of MSCs cultured on plastic surface (Ctrl), FBS coating and (FBS) or platelet lysate coating (hPLP) on day 12.

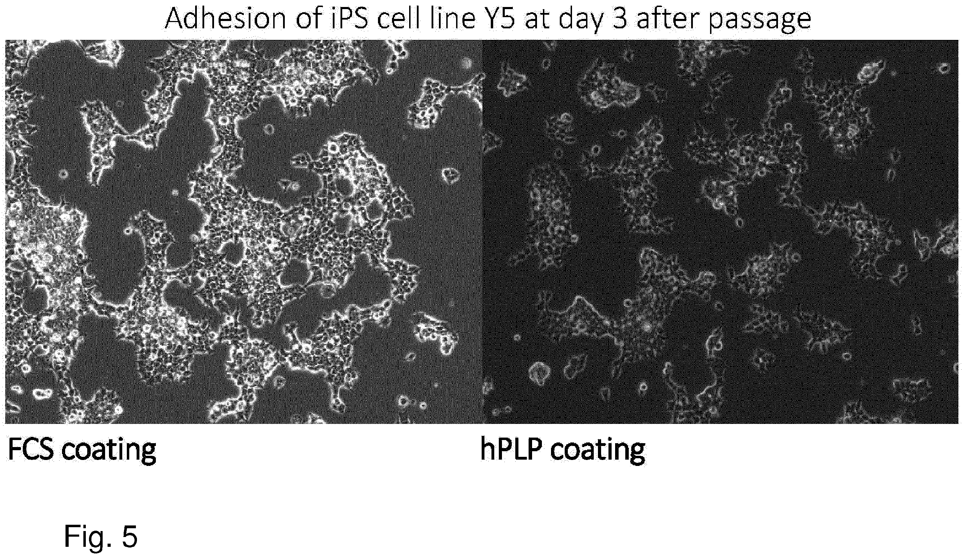

- FIG. 5: shows the cell numbers from iPSC and fold increase of the cell numbers of iPSCs cultured on iHFF (HFF), Matrigel (M) or platelet lysate coating (hPLP) on day 5 after enzymatic passage.
iPSC cultured on the hPLP thin film express multiple pluripotency markers consistent with an undifferentiated phenotype, including alkaline phosphatase, Oct4, Nanog, SSEA3, and Sox2, TRA181, SSEA4 and TRA160. Both cell lines demonstrate uniform expression of markers of pluripotency by immunocytochemistry after extended culture in the hPLP coating system. qPCR analyses confirm the pluripotent status hES cell cultures. Expression of standard pluripotency markers in the hPLP coating system is comparable to that demonstrated within the traditional feeder based culture (HFF) and with the Matrigel system. In addition, there is no clear variation of expression between early passage (P3) and later passage (P13). The cells cultured in the hPLP coating system do not express markers associated with differentiation after extended culture periods. β-III tubulin, SSEA1, Sox17, FoxA2 and α-smooth muscle actin (ASMA) were not detected when analyzed by immunocytochemistry and BRACHYURY (T), SOX17, GSC, NKX2.5, CDX2, EOMES, SOX7 and PAX6 were not induced compared to control (HFF culture and Matrigel culture) levels when analyzed by qPCR.

### METHODS IN DETAIL

### Cell Culture on hPLP thin film

### Preparation of the hPLP thin film as culture vessel matrix

250 μl of hPLP per cm² were added to a culture dish made of hydrophobic Polystyrene, the solution was incubated in a humidified incubator at 37 °C in the presence of 5 % CO² 95 % air for 1/2 hours. The hPLP solution was removed and was washed by once by PBS.

Optionally, the hPLP film is completely air-dryed slowly in an aseptic manner in a laboratory class II cabinet. The dehydrated film is hydrated by adding 250 μl of PBS for 15 min and can be incubated with cells.

### Proliferation Experiments

MSCs derived from bone marrow (passage 3) were used for *in vitro* expansion in DMEM supplemented with 10 % FBS, 10 % FBS. Cells were seeded in a density of 50-100 cells/cm², medium was changed twice weekly. On day 7 cells were harvested using TriPLE Select and the number of living cells was calculated after staining the cells with Trypan Blue which stains the dead cells. All cell numbers given refer to the number of living cells.

### Medium iPSC, MSCs

Preferably, the medium for MSCs is DMEM. This medium is characterized by the presence of 10 % FCS, lipoic acid, sodium pyruvate, ascorbic acid and vitamin B12. The medium may be purchased from companies such as Lonza, Cambrex, Invitrogen, Sigma-Aldrich and Stem Cell Technologies.

Although the use of the medium of the present invention is described in detail for the culturing of MSCs, it is also suitable for culturing other cells, in particular the growth of cells that depend on growth factors released by the platelets, e.g. endothelial cells such as HUVECs, endothelial progenitor cells (EPC) and endothelial stem cells (ESC), fibroblasts, osteoblasts, keratinocytes, cartilage cells, and human stem cells.

Preferred cell types which may be cultured on the thin film substrate in accordance with the invention are human stem cells (hES) and progenitor cells.

The term "stem cells" refers to cells, which have retained the capacity to proliferate and differentiate into different cell types. Stem cells in accordance with the present invention can comprise multipotent, pluripotent and totipotent stem cells.

Totipotent stem cells still have the capacity to differentiate into all different cell lineages of a whole organism and thus may give rise to a complete organism. Pluripotent stem cells have retained the capacity to differentiate in the three germ layers endoderm, ectoderm and mesoderm. Multipotent stem cells are restricted to a limited range of cell types and cannot differentiate into the three germ layers.

In a preferred embodiment, stem cells will be neural stem cells, mesenchymal stem cells, endothelial stem cells, haematopoietic stem cells or epithelial stem cells. A particularly preferred cell type are mesenchymal stem cells.

Mesenchymal stem cells, which are also known as marrow stromal cells or mesenchymal progenitor cells, are defined as self-renewable, multi-potent progenitor cells with a capacity to differentiate into several distinct mesenchymal lineages. MSCs have been demonstrated to differentiate into lineage-specific cells that form bone, cartilage, fat, muscle tissue and tendon. Their surface phenotype has been defined as being negative for CD45 and CD34 and positive for CD73, CD90, and CD105. They can be isolated from the cord blood or bone marrow.

The differentiation of MSCs to the chondrogenic lineage can be initiated by the treatment of the cells with TGF-1, whereas the osteogenic linneage is initiated by dexamethasone etc. Differentiating the MSCs to the osteogenic or the adipogenic lineage:

The induction of the chondrogenic differentiation can be detected by staining the MSC derived chondrogenic cells with Safranin O after incubation with chondrogenesis-inducing culture medium. The osteogenic differentiation can be detected by a positive reaction to alkaline phosphatase and von Kossa silver staining after induction with medium containing osteo-inductive components.

The "progenitor cells" are early descendants of stem cells, which may lose their ability of self-renewal. The progenitor cells are able to differentiate to different cell types within one germ layer, but in contrast to stem cells, they cannot differentiate to cells of a different germ layer. There are three different germ layers, i. e. endoderm, ectoderm and mesoderm. The endoderm is the internal cell layer of the embryo from which the lung, digestive tract, bladder and urethra are formed. The ectoderm is the surface layer of the embryo that develops into the epidermis, skin, nerves, hair, etc. The mesoderm is the middle cell layer of the embryo from which the connective tissue, muscles, cartilage, bone, lymphoid tissues, etc are formed.

The invention is based on the important finding that a plasma-free platelet lysate thin film coat improves significantly the adhesion and the proliferation of cells, preferably of iPS cells and MSCs and therefore may be used as a cell culture matrix avoiding the potential negative.

## Claims

1. A process of improving the expansion of eukaryotic cells in culture in an aqueous medium which is contained in a culture vessel comprising a substrate for the eukaryotic cells, the process comprising the steps of:
- providing a lysate obtainable from a thrombocytes containing source,
- incubating the substrate with the lysate for a sufficient time to allow a coating of the substrate with components of the lysate prior to
- contacting the eukaryotic cells with the substrate.

2. The process of claim 1 wherein the substrate is provided by an inner surface of the culture vessel, the substrate is a scaffold, a micro-carrier or a vessel.

3. The process of claim 1 or 2 wherein the culture vessel is a flask, bioreactor or tube.

4. The process of any one of the claims 1 to 4 wherein the eukaryotic cells are selected from the group consisting of stem cells, human embryonic stem cell Induced Pluripotent Stem cells (iPS), HUFS or progenitor cells.

5. The process of any one of the claims 1 to 4 wherein the medium is stem cell or cell culture medium.

6. The process of any one of the claims 1 to 5 wherein the time to allow a coating of the substrate is at least about ten seconds, in particular about one hour to about 48 hours.

7. The process of any one of the claims 1 to 6 wherein the components of the lysate comprise substances are selected of the group consisting of laminin, fibronectin, collagens and other glycoproteins, hyaluronic acid, proteoglycans, and elastins.

8. A process for manufacturing a coated substrate comprising the steps of
- providing the substrate,
- contacting the substrate with a lysate of thrombocytes in a medium sufficient to coat the substrate with components of the lysate of thrombocytes,
- removing the medium.

9. The process of claim 8 wherein after removing of the medium a finishing step is performed, such as washing steps, deep-freezing or freeze-drying, or air drying.

10. The substrate of claim 9 wherein the substrate is a coated microcarrier, coated scaffold, culture vessel having a coated inner surface.
